# FASCICULE DE BREVET EUROPEEN

(11) **EP 1 059 099 B1**
(45) Date de publication et mention de la délivrance du brevet: **12.05.2004**
(21) Numéro de dépôt: 00401605.1
(22) Date de dépôt: 07.06.2000
(51) Int. Cl.: A61N 1/368

(54) **Stimulateur cardiaque de type multisite, comportant des moyens de stimulation resynchronisée pour le traitement de l'insuffisance cardiaque**
Gerät zur Stimulation mehrerer Herzbereiche mit Resynchronisationsvorrichtung zur Behandlung von Herzinsuffizienz
Multisite cardiac stimulator with resynchronization means for treatment of cardiac insufficiency

(30) Priorité: 11.06.1999 FR 9907388
(43) Date de publication de la demande: 13.12.2000
(73) Titulaire: ELA MEDICAL (Société anonyme), 92541 Montrouge (FR)
(72) Inventeur: Ripart, Alain, 91170 Gif sur Yvette (FR); Bouhour, Anne, 92410 Ville d'Avray (FR)
(74) Mandataire: Dupuis-Latour, Dominique

(56) Documents cités:
- US-A- 4 344 437
- US-A- 4 945 909
- US-A- 5 074 304

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement les prothèses dites "multisite" dans lesquelles des électrodes sont placées en une pluralité de sites respectifs distincts comportant au moins un site ventriculaire et un site auriculaire. Il peut s'agir d'une prothèse de type "double chambre" (stimulation atriale droite et stimulation ventriculaire droite) ou, le plus souvent, "triple chambre" (stimulation atriale droite et double stimulation ventriculaire) ou "quadruple chambre" (double stimulation atriale et double stimulation ventriculaire).

En effet, outre le traitement des troubles du rythme cardiaque, il a été proposé de traiter par stimulation les troubles de contraction myocardique observés chez des patients en insuffisance cardiaque, ces troubles pouvant être spontanés ou induits par une stimulation traditionnelle. On pourra notamment se référer à l'étude de J. C. Daubert et coll., *Stimucoeur,* 25, n°3, pp. 170-176 qui fait le point des travaux sur ce sujet.

Il a été notamment proposé de stimuler simultanément les ventricules gauche et droit, en permanence, pour les resynchroniser, ce qui a permis d'observer des résultats souvent spectaculaires pour des patients en insuffisance cardiaque en classe III, non améliorée par les traitements classiques.

Le patient retire d'un tel traitement un bénéfice instantané se traduisant par une sensation de revivre, qui l'entraîne à reprendre peut-être des activités jusqu'alors devenues impossibles.

Cependant, son système cardio-vasculaire n'a pas eu le temps de se réadapter à cette nouvelle situation de sorte que, du fait de la reprise d'une vie presque normale, le patient peut solliciter trop violemment un coeur fatigué et malade.

Or, dans ces circonstances, le médecin ne peut ni limiter la capacité d'effort du patient pendant une durée trop importante, ce qui réduirait l'efficacité du dispositif, ni revoir son patient à des intervalles dé temps trop rapprochés.

L'un des buts de l'invention est de remédier aux difficultés entraînées par la situation décrite plus haut, en offrant au patient une capacité d'effort progressive, évolutive de façon automatique au cours du temps.

Essentiellement, l'invention propose d'adapter le paramètre connu sous l'appellation "fréquence maximale" ou "Fmax", qui est la fréquence maximale de suivi du rythme atrial par le ventricule, c'est-à-dire la limite supérieure à laquelle le stimulateur peut synchroniser une stimulation ventriculaire sur chaque détection auriculaire en mode DDD.

Ce paramètre Fmax sert notamment à plafonner la fréquence de stimulation calculée par des algorithmes tels que les fonctions de lissage ou d'asservissement.

Dans un stimulateur double chambre, la fréquence maximale est également utilisée comme valeur de référence, comparée à la fréquence atriale détectée afin de limiter la fréquence de stimulation du (des) ventricule(s) lorsque le rythme atrial dépasse la valeur de la fréquence maximale, par exemple en appliquant un mode de fonctionnement dit "mode Wenckebach".

La fréquence maximale était jusqu'à présent programmée une fois pour toutes à une valeur déterminée, principalement en fonction de l'âge du patient, éventuellement pondéré par la capacité d'effort de celui-ci et/ou la présence d'une cardiopathie ou cardiomyopathie.

L'invention propose un dispositif du type de type multisite connu comportant des moyens de stimulation resynchronisée pour le traitement de l'insuffisance cardiaque, caractérisé en ce qu'il comprend des moyens aptes à faire croître progressivement au cours du temps la fréquence maximale de stimulation, à partir d'une valeur d'origine jusqu'à une valeur cible, la fréquence maximale étant notamment accrue à intervalles réguliers par incréments successifs.

Dans une forme de mise en oeuvre avantageuse, lorsque le dispositif comporte des moyens pour raccourcir le délai atrio-ventriculaire lorsqu'augmente la fréquence cardiaque instantanée, le dispositif comporte alors en outre des moyens aptes à comparer la fréquence cardiaque instantanée à un seuil prédéterminé, et aptes à ne permettre la réduction du délai atrio-ventriculaire que lorsque la fréquence cardiaque instantanée est située en deçà de ce seuil. Dans le cas contraire, il est avantageusement prévu des moyens aptes à allonger progressivement le délai atrio-ventriculaire lorsqu'augmente la fréquence cardiaque instantanée. Très avantageusement, il est prévu des moyens aptes à faire croître progressivement au cours du temps la valeur dudit seuil prédéterminé.

On va maintenant décrire un exemple de mise en oeuvre de l'invention, en référence aux dessins annexés.

La figure 1 montre l'évolution, en fonction du temps, de la fréquence maximale ajustée selon l'invention.

Les figures 2 à 4 illustrent la variation en phase d'effort du délai atrio-ventriculaire (DAV) en fonction de la fréquence cardiaque instantanée, à trois stades différents.

Au départ, lorsque le médecin programme l'appareil de manière à mettre en place une stimulation ventriculaire resynchronisée, en plus des paramètres habituels il programme un certain nombre d'autres paramètres permettant la mise en oeuvre de l'invention, qui sont :
- une fréquence maximale de départ Fₒ,
- une fréquence maximale optimale F₁, qui sera considérée comme une valeur cible à atteindre, fonction de l'état du patient, et
- le temps au bout duquel cette fréquence optimale devra être atteinte.

Il peut ainsi, par exemple, programmer une fréquence maximale de départ de 100 cpm et une fréquence maximale optimale de 140 cpm, à atteindre au bout d'un mois.

Le logiciel de commande du stimulateur va alors automatiquement adapter la fréquence maximale afin d'atteindre la valeur cible F₁ au bout du temps considéré, à partir de la fréquence d'origine Fₒ initiale.

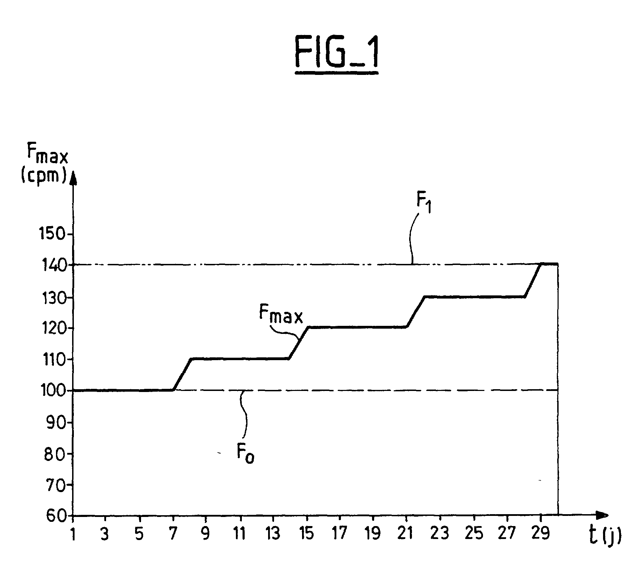

Cette adaptation progressive peut se faire notamment, comme illustré sur la figure 1 correspondant aux valeurs de l'exemple numérique donné plus haut, par incréments progressifs, par exemple de 10 cpm tous les 7 jours.

Les conséquences physiologiques de cet ajustement de la fréquence maximale sont les suivantes.

Pour un patient n'ayant pas de rythme propre (par exemple un patient en fibrillation auriculaire), sa capacité d'effort sera naturellement limitée par sa fréquence cardiaque.

Pour un patient en rythme sinusal avec conduction atrio-ventriculaire spontanée, la fréquence cardiaque ne sera pas limitée, mais la resynchronisation s'arrêtera au-dessus de la fréquence maximale lorsque celle-ci sera atteinte. L'invention s'adresse, dans ce cas, à des patients très symptomatiques, qui arrêteront immédiatement l'effort et limiteront par-là leur activité. Pour ce dernier type de patient, cette approche pourra également être complétée par un traitement anti-arythmique médicamenteux, qui aura pour effet, de limiter l'accélération sinusale.

Une autre possibilité de limitation de la capacité d'effort du patient consiste, de façon également caractéristique de l'invention, à adapter le délai atrio-ventriculaire (DAV), de la manière que l'on va maintenant exposer.

Pour la stimulation ventriculaire resynchronisée, le DAV est habituellement ajusté de manière à capturer 100 % des dépolarisations ventriculaires. De façon générale, le DAV varie en fonction de la fréquence cardiaque instantanée, en diminuant lorsque cette fréquence augmente.

On notera que la fréquence instantanée du patient est soit une fréquence spontanée - sinusale - soit une fréquence stimulée, la fréquence de stimulation étant dans ce cas gérée par le stimulateur entre fréquence de base et fréquence maximale, en fonction de l'état d'effort du patient.

Au moment où la stimulation resynchronisée est mise en place par le médecin, celui-ci programme, outre les paramètres habituels, une valeur que l'on appellera "fréquence maximale pour l'adaptation du DAV", désignée ci-après "Fmax_DAVauto", avec une valeur de départ de cette fréquence. Il programme également la fréquence maximale du patient, ainsi que le temps au bout duquel il souhaite que Fmax_DAVauto atteigne cette fréquence maximale.

Par exemple, le médecin programme une Fmax_DAVauto de départ de 100 cpm et une fréquence maximale de 140 cpm à atteindre au bout d'un mois.

L'algorithme de gestion des fonctions du stimulateur va alors adapter automatiquement la valeur de Fmax_DAVauto en la faisant croître, dans l'exemple cité, de 10 cpm tous les sept jours.

Cette valeur, évolutive au cours du temps, de Fmax_DAVauto va constituer un seuil commandant la manière dont le DAV va être modifié en fonction des variations de la fréquence cardiaque instantanée.

Tant que la fréquence cardiaque instantanée reste inférieure au seuil Fmax_DAVauto, le DAV est modifié de la manière habituelle, c'est-à-dire qu'il est automatiquement raccourci lorsque la fréquence cardiaque instantanée augmente (le DAV variant entre des bornes préprogrammées DAVmin et DAVmax).

En revanche, dès que la fréquence cardiaque instantanée passe au-dessus de Fmax_DAVauto, le DAV va s'allonger progressivement, par exemple de 10 ms tous les quatre cycles cardiaques (ces valeurs étant paramétrables), jusqu'à détecter une dépolarisation ventriculaire ou bien jusqu'à une valeur limite DAVmax.

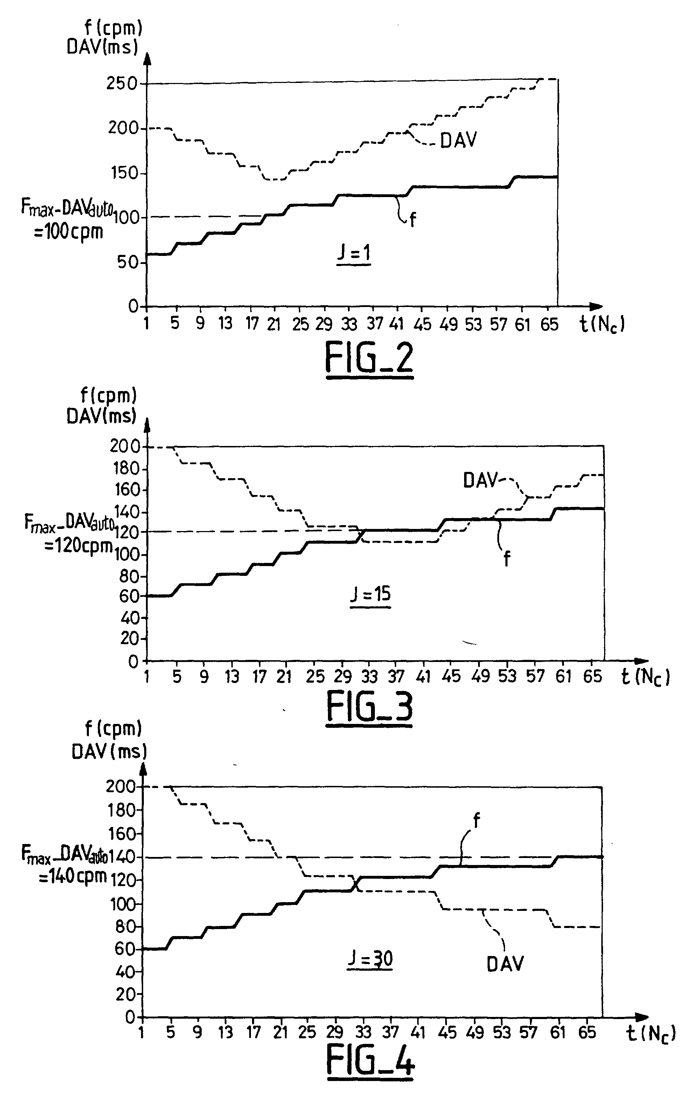

Les figures 2 à 4 illustrent la manière dont évolue dans cet exemple le délai atrio-ventriculaire DAV (en tiretés, exprimé en millisecondes) en fonction de la fréquence cardiaque instantanée f (en trait plein, exprimée en cpm) dans une phase d'effort (le temps en abscisse étant exprimé en nombre N_{c} de cycles cardiaques successifs).

On notera que pour mieux faire ressortir la spécificité de cette caractéristique, on a pris l'hypothèse d'une valeur constante de Fmax, égale dès le départ à la valeur optimale cible, en gardant néanmoins à l'esprit qu'en pratique les deux paramètres Fmax et Fmax_DAVauto peuvent évoluer l'un et l'autre progressivement au cours du temps.

Les figures montrent la manière dont varie le DAV en fonction de la fréquence cardiaque à trois stades :
- figure 2 : au premier jour, lorsque Fmax_DAVauto = 100 cpm ;
- figure 3 : au quinzième jour, lorsque Fmax_DAVauto = 120 cpm ;
- figure 4 : au trentième jour, lorsque Fmax _DAVauto = 140 cpm.

Dans ces exemples, l'effort est le même dans les trois cas, et par ailleurs le DAV est en tout état de cause borné aux deux valeurs DAVmin = 80 ms et DAVmax = 200 ms.

## Revendications

1. Un dispositif médical implantable actif, notamment un stimulateur cardiaque, défibrillateur et/ou cardioverteur de type multisite, comportant des moyens de stimulation resynchronisée pour le traitement de l'insuffisance cardiaque,
**caractérisé en ce qu'**il comprend des moyens aptes à faire croître progressivement au cours du temps la fréquence maximale de stimulation, à partir d'une valeur d'origine jusqu'à une valeur cible.

2. Le dispositif de la revendication 1 dans lequel la fréquence maximale est accrue à intervalles réguliers par incréments successifs.

3. Le dispositif de la revendication 1 dans lequel, le dispositif comportant des moyens pour raccourcir le délai atrio-ventriculaire lorsqu'augmente la fréquence cardiaque instantanée, le dispositif est **caractérisé en ce qu'**il comporte des moyens aptes à comparer la fréquence cardiaque instantanée à un seuil prédéterminé, et aptes à ne permettre la réduction du délai atrio-ventriculaire que lorsque la fréquence cardiaque instantanée est située en deçà de ce seuil.

4. Le dispositif de la revendication 3, comportant des moyens aptes à allonger progressivement le délai atrio-ventriculaire lorsqu'augmente la fréquence cardiaque instantanée, lorsque cette fréquence cardiaque instantanée est située au-delà dudit seuil.

5. Le dispositif de la revendication 3, comprenant en outre des moyens aptes à faire croître progressivement au cours du temps la valeur dudit seuil prédéterminé.

## Patentansprüche

1. Aktive, implantierbare medizinische Vorrichtung, insbesondere ein Herzschrittmacher, Defibrillator und/oder Kardiovertierer eines verteilten Typs, welche Mittel zur resynchronisierten Stimulation für die Behandlung von Herzfehlern umfasst,
**dadurch gekennzeichnet, dass**
sie Mittel umfasst, die geeignet sind, progressiv im Verlauf über die Zeit die maximale Frequenz der Stimulation zu erhöhen, ausgehend von einem Ursprungswert bis zu einem Zielwert.

2. Vorrichtung nach Anspruch 1, worin die maximale Frequenz zu regelmäßigen Intervallen durch aufeinander folgende Inkremente erhöht wird.

3. Vorrichtung nach Anspruch 1, worin die Vorrichtung aufweist Mittel zum Verkürzen der atrio-ventrikulären Verzögerung beim Erhöhen der momentanen Herzfrequenz, wobei die Vorrichtung **dadurch gekennzeichnet ist, dass** sie dazu geeignete Mittel umfasst, die momentane Herzfrequenz mit einer vorherbestimmten Schwelle zu vergleichen, und dazu geeignet, die Reduzierung der atrio-ventrikulären Verzögerung nicht zu erlauben, bis die momentane Herzfrequenz diesseits dieser Schwelle liegt.

4. Vorrichtung nach Anspruch 3, welche Mittel umfasst, die geeignet sind, die atrio-ventrikuläre Verzögerung progressiv zu verlängern beim Steigen der momentanen Herzfrequenz, wenn diese momentane Herzfrequenz jenseits der Schwelle liegt.

5. Vorrichtung nach Anspruch 3, weiterhin aufweisend geeignete Mittel zum progressiven Erhöhen über die Zeit des Werts der vorherbestimmten Schwelle.

## Claims

1. An active implantable medical device, in particular a cardiac pacemaker, defibrillator and/or cardioverter of the multisite type, comprising means for resynchronised stimulation for the treatment of cardiac insufficiency,
**characterised by** including means adapted to progressively increase over time the maximal stimulation frequency, from an initial value to a target value.

2. The device of claim 1, wherein the maximal stimulation frequency is increased at regular intervals by successive increments.

3. The device of claim 1, wherein, the device including means for shortening the atrio-ventricular delay as the current heart rate increases, the device is **characterised by** including means for comparing the current heart rate with a predetermined threshold, and adapted to allow the shortening of the atrio-ventricular delay only when the current heart rate is below said threshold.

4. The device of claim 3, including means adapted to progressively increase the atrio-ventricular delay as the current heart rate increases, when said current heart rate stands beyond said predetermined threshold.

5. The device of claim 3, further including means adapted to progressively increase over time the value of said predetermined threshold.
